(19) **Europäisches Patentamt**
**European Patent Office**
**Office européen des brevets**

(11) **EP 3 871 154 B1**

(12) **EUROPEAN PATENT SPECIFICATION**

(45) Date of publication and mention
of the grant of the patent:
**23.07.2025 Bulletin 2025/30**

(21) Application number: **19875291.7**

(22) Date of filing: **22.10.2019**

(51) International Patent Classification (IPC):
**G06N 3/084** (2023.01)     **G06N 20/10** (2019.01)
**G06N 20/20** (2019.01)     G06N 3/045 (2023.01)
**G16C 20/70** (2019.01)     G16C 20/30 (2019.01)

(52) Cooperative Patent Classification (CPC):
**G16C 20/30; G06N 3/084; G06N 20/10;**
**G06N 20/20;** G06N 3/045; G16C 20/70

(86) International application number:
**PCT/US2019/057468**

(87) International publication number:
**WO 2020/086604 (30.04.2020 Gazette 2020/18)**

(54) **SYSTEMS AND METHODS FOR ACTIVE TRANSFER LEARNING WITH DEEP FEATURIZATION**

SYSTEME UND VERFAHREN ZUM AKTIVEN TRANSFERLERNEN MIT TIEFER FEATURISIERUNG

SYSTÈMES ET PROCÉDÉS D'APPRENTISSAGE PAR TRANSFERT ACTIF AVEC
CARACTÉRISATION PROFONDE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **23.10.2018 US 201862749653 P**

(43) Date of publication of application:
**01.09.2021 Bulletin 2021/35**

(73) Proprietor: **The Board of Trustees of the Leland**
**Stanford**
**Junior University**
**Stanford, CA 94305-2038 (US)**

(72) Inventors:
• **FEINBERG, Evan, N.**
**Stanford, CA 94305 (US)**
• **PANDE, Vijay, S.**
**Stanford, CA 94305 (US)**

(74) Representative: **Patentanwaltskanzlei**
**Matschnig & Forsthuber OG**
**Biberstraße 22**
**Postfach 36**
**1010 Wien (AT)**

(56) References cited:
US-A1- 2016 034 809     US-A1- 2017 161 635
US-A1- 2018 204 111     US-B1- 8 818 910
US-B2- 9 779 081

• **DEHZANGI ABDOLLAH ET AL: "A Combination**
**of Feature Extraction Methods with an Ensemble**
**of Different Classifiers for Protein Structural**
**Class Prediction Problem", IEEE/ACM**
**TRANSACTIONS ON COMPUTATIONAL**
**BIOLOGY AND BIOINFORMATICS, IEEE**
**SERVICE CENTER, NEW YORK, NY, US, vol. 10,**
**no. 3, 1 May 2013 (2013-05-01), pages 564 - 575,**
**XP011566568, ISSN: 1545-5963, [retrieved on**
**20141205], DOI: 10.1109/TCBB.2013.65**
• **HUSSEIN HIJAZI ET AL: "Ensemble**
**Classification of Cancer Types and Biomarker**
**Identification", DRUG DEVELOPMENT**
**RESEARCH, NEW YORK, NY, US, vol. 73, no. 7,**
**18 October 2012 (2012-10-18), pages 414 - 419,**
**XP071572498, ISSN: 0272-4391, DOI: 10.1002/**
**DDR.21032**
• **HONGMING CHEN ET AL: "The rise of deep**
**learning in drug discovery", DRUG DISCOVERY**
**TODAY, vol. 23, no. 6, 31 January 2018**
**(2018-01-31), AMSTERDAM, NL, pages 1241 -**
**1250, XP055664738, ISSN: 1359-6446, DOI:**
**10.1016/j.drudis.2018.01.039**

- EVAN N FEINBERG ET AL: "Spatial Graph Convolutions for Drug Discovery", ARXIV.ORG, CORNELL UNIVERSITY LIBRARY, 201 OLIN LIBRARY CORNELL UNIVERSITY ITHACA, NY 14853, 12 March 2018 (2018-03-12), XP080863870

- JING ET AL. ET AL.: "Deep Learning for Drug Design: an Artificial Intelligence Paradigm for Drug Discovery in the Big Data Era", AAPS J., vol. 20, no. 3, 30 March 2018 (2018-03-30), pages 58, XP036534353

# EP 3 871 154 B1

**Description**

FIELD OF THE INVENTION

The present invention generally relates to learning for machine learning models and more specifically relates to active transfer learning with deep featurization.

BACKGROUND

Supervised machine learning (ML) is an umbrella term for a family of functional forms and optimization schemes for mapping input features representing input samples to ground truth output labels. Deep neural networks (DNN) denote a set of functional forms which frequently surpass previous generations of ML methods by learning the features pertinent to the prediction task at hand in intermediate neural network layers.

Deep neural networks frequently surpass their predecessors by employing feature learning instead of feature engineering. Traditional supervised machine learning (ML) techniques train models that map fixed, often hand-crafted, features to output labels. In contrast, deep neural networks often take as input a more elementary featurization of the input - grids of pixels for images, one-hot encoded words for natural language - and "learn" the features most immediately relevant to the task at hand in the intermediate layers of the neural network. Efficient means for training neural networks can be difficult to identify, particularly across different fields and applications.

Dehzangi Abdollah et al.: "A Combination of Feature Extraction Methods with an Ensemble of Different Classifiers for Protein Structural Class Prediction Problem", IEEE/ACM Transactions On Computational Biology And Bioinformatics, IEEE Service Center, New York, NY, US, vol. 10, no. 3, 1 May 2013, pages 564-575, describe that better understanding of structural class of a given protein reveals important information about its overall folding type and its domain and that it can also be directly used to provide critical information on general tertiary structure of a protein which has a profound impact on protein function determination and drug design. Dehzangi et al. further describe that, despite tremendous enhancements made by pattern recognition-based approaches to solve this problem, it still remains as an unsolved issue for bioinformatics which demands more attention and exploration. Dehzangi et al. propose a feature extraction model which incorporates physicochemical and evolutionary-based information simultaneously. Dehzangi et al. further propose overlapped segmented distribution and autocorrelation based feature extraction methods to provide more local and global discriminatory information. Proposed feature extraction methods are explored for 15 most promising attributes that are selected from a wide range of physicochemical-based attributes. An ensemble of different classifiers is applied, and enhancement of the protein structural class prediction accuracy for four popular benchmarks is shown.

SUMMARY OF THE INVENTION

Systems and methods for active transfer learning in accordance with independent claims 1 and 15, respectively, are illustrated. The method includes training a deep featurizer. The method includes steps for training a master model and a set of one or more secondary models, wherein the master model includes a set of one or more layers, freezing weights of the master model, generating a set of one or more outputs from the master model, and training a set of one or more orthogonal models on the generated set of outputs.

In a further embodiment, training the master model includes training the master model for several epochs.

Each epoch includes training the master model and the set of secondary models on several datasets.

In a still further embodiment, generating the set of one or more outputs includes propagating the several datasets through the master model.

Each dataset of the several datasets has labels for a different characteristic of inputs of the dataset.

In a yet further embodiment, the method further includes steps for validating the master model and the set of orthogonal models.

In another additional embodiment, validating the set of orthogonal models includes computing an out of bag score for the set of orthogonal models.

In a further additional embodiment, validating the set of orthogonal models comprises training the master model on a master data set includes a training data set and a validation data set, training the set of orthogonal models on the training data set, and computing a validation score for the orthogonal models based on the validation data set.

In a further embodiment again, the set of orthogonal models includes at least one of a random forest and a support vector machine.

In still yet another embodiment, training the master model comprises training the master model for a plurality of epochs, wherein the method further includes steps for, for each particular orthogonal model, identifying an optimal epoch of the plurality of epochs by validating the master model and the particular orthogonal model. The method further includes steps for compositing the master model and the particular orthogonal model at the optimal epoch as a composite model to classify a new set of inputs.

In a still yet further embodiment, at least one secondary model of the set of secondary models is a neural network includes a set of one or more layers.

One embodiment includes a non-transitory machine readable medium containing processor instructions accordance with independent claim 14.

A computer-implemented method for drug discovery in accordance with claim 1 is provided.

In a still further embodiment, prior to creating a set of one or more outputs, the method comprises freezing weights of the master model.

In another additional embodiment, the set of orthogonal models includes at least one of random forest, a support vector machine, XGBoost, linear regression, nearest neighbor, naïve bayes, decision trees, neural networks, and k-means clustering.

In a further additional embodiment, the method further includes steps for compositing the master model and the set of orthogonal models as a composite model to classify a new set of inputs.

In another embodiment again, the method further includes steps for, prior to training a deep featurizer, preprocessing the one or more datasets of one or more molecules.

In a further embodiment again, preprocessing the one or more datasets further includes at least one of the following formatting, cleaning, sampling, scaling, decomposing, converting data formats, or aggregating.

In still yet another embodiment, the trained master model or trained orthogonal model predicts a property of the drug candidate.

In a still yet further embodiment, the property of the drug candidate includes at least one of the group consisting of absorption, distribution, metabolism, elimination, toxicity, solubility, metabolic stability, in vivo endpoints, ex vivo endpoints, molecular weight, potency, lipophilicity, hydrogen bonding, permeability, selectivity, pKa, clearance, half-life, volume of distribution, plasma concentration, and stability.

In still another additional embodiment, the one or more molecules is a ligand molecule and/or a target molecule.

In a still further additional embodiment, the target molecule is a protein.

In still another embodiment again, the method further includes steps for preprocessing the one or more datasets.

In a still further embodiment again, preprocessing the one or more datasets further includes at least one of the following formatting, cleaning, sampling, scaling, decomposing, converting data formats, or aggregating.

The method further includes steps for, prior to identifying the drug candidate, creating a feature set of one or more outputs from the deep featurizer.

The method further includes steps for using the trained orthogonal model on the feature set to identify the drug candidate.

One embodiment includes a system for drug discovery comprising one or more processors in accordance with independent claim 15.

In another embodiment, prior to creating a set of one or more outputs from the master model, the one or more processors are further configured to freeze weights of the master model.

In yet another embodiment, the one or more processors are individually or collectively configured to train the master model for one or more epochs.

In yet another embodiment again, training the master model for each epoch includes training the master model and the set of secondary models on one or more datasets.

In a yet further embodiment again, creating the set of one or more outputs includes propagating the one or more datasets through the master model.

Each dataset of the one or more datasets has labels for a different characteristic of inputs of the dataset.

In a further additional embodiment again, the one or more processors are further configured to validate the master model and the set of orthogonal models.

In still yet another additional embodiment, validating the set of orthogonal models includes computing an out of bag score for the set of orthogonal models.

In a further embodiment, validating the set of orthogonal models comprises training the master model on a master data set that includes a training data set and a validation data set, training the set of orthogonal models on the training data set, and computing a validation score for the orthogonal models based on the validation data set.

In a still further embodiment, the set of orthogonal models includes at least one of random forest, a support vector machine, XGBoost, linear regression, nearest neighbor, naïve bayes, decision trees, neural networks, and k-means clustering.

In yet another embodiment, the one or more processors are further configured to composite the master model and the set of orthogonal models as a composite model to classify a new set of inputs.

In a yet further embodiment, prior to training a deep featurizer, the one or more processors are further configured to preprocess the one or more datasets of one or more molecules.

In another additional embodiment, preprocessing the one or more datasets further includes at least one of the following formatting, cleaning, sampling, scaling, decomposing, converting data formats, or aggregating.

In a further additional embodiment, the trained master model or trained orthogonal model is configured to predict a property of the drug candidate.

In another embodiment again, the property of the drug candidate includes at least one of the group consisting of absorption, distribution, metabolism, elimination, toxicity, solubility, metabolic stability, in vivo endpoints, ex vivo endpoints, molecular weight, potency, lipophilicity, hydrogen bonding, permeability, selectivity, pKa, clearance, half-life, volume of distribution, plasma concentration, and stability.

In a still yet further embodiment, the one or more processors are further configured to preprocess the one or more datasets.

In still another additional embodiment, the one or more processors that are individually or collectively configured to preprocess the one or more datasets further includes at least one of the following formatting, cleaning, sampling, scaling, decomposing, converting data formats, or aggregating.

Prior to identifying the drug candidate, the one or more processors are further configured to create a feature set of one or more outputs from the deep featurizer.

In still another embodiment again, the one or more processors are further configured to use the trained orthogonal model on the feature set to identify the drug candidate.

Additional embodiments and features are set forth in part in the description that follows, and in part will become apparent to those skilled in the art upon examination of the specification or may be learned by the practice of the invention. A further understanding of the nature and advantages of the present invention may be realized by reference to the remaining portions of the specification and the drawings, which forms a part of this disclosure.

BRIEF DESCRIPTION OF THE DRAWINGS

The description and claims will be more fully understood with reference to the following figures and data graphs, which are presented as exemplary embodiments of the invention and should not be construed as a complete recitation of the scope of the invention as defined by the appended claims.

Figure 1 illustrates an example of a method for active transfer learning with deep featurization.

Figures 2 and 3 illustrate an active transfer learning process in accordance with an embodiment of the invention.

Figure 4 illustrates a system that trains machine learning models in accordance with some embodiments of the invention.

Figure 5 illustrates an example of a model training element that executes instructions to perform processes that train master and/or orthogonal models.

Figure 6 illustrates an example of a training application for providing training tasks in accordance with an embodiment of the invention.

DETAILED DESCRIPTION

Turning now to the drawings, systems and methods for training deep featurizers are described below. In certain embodiments, deep featurizers are neural networks, such as (but not limited to) convolutional neural networks and graph convolutional networks, which can be used to identify features from an input. Deep featurizers (or master models) can be trained with classifiers (or secondary models) to predict labels for a given input and to train the deep featurizer (e.g., through backpropagation) to identify features relevant to a given label. Deep featurizers in accordance with various embodiments of the invention are trained with multiple different data sets associated with multiple different labels to train a single deep featurizer to identify features that are more generally useful for identifying the different labels for the inputs. Deep featurizers are further trained with orthogonal models that train on intermediate outputs (e.g., the penultimate fully connected layer) of the deep featurizers and/or classifiers. Orthogonal models in accordance with some embodiments of the invention do not share gradient information with the master model, and can include non-differentiable and/or ensemble models, such as (but not limited to) random forests and support vector machines. In some embodiments, orthogonal models can be used to classify inputs, as well as to validate the performance of deep featurizers. Such systems of deep featurizers, classifiers and orthogonal models can allow for efficient training of the models, while avoiding overfitting to any particular data set. In addition, training in such a manner in accordance with many embodiments of the invention can allow for efficient and effective training of models using one or more data sets that can have varying degrees of overlap.

For example, in pharmaceutical development, chemists have access to data sets that each map molecular structures to at least one chemical property of interest. For instance, a chemist may have access to a database of 10,000 chemicals and associated hepatoxicity outcomes, 15,000 chemicals and associated LogD measurements, 25,000 chemicals and associated passive membrane permeability measurements, etc. There is often varying degrees of overlap between such data sets. Methods in accordance with various embodiments of the invention can leverage all of the chemical data to which one has access, in order to build superior deep learning models for all tasks of interest that can exceed the performance of training separate models for each data set individually. Technical problems in the context of chemical property prediction can arise from a relative paucity of available, high-quality, labeled training data for a given set of characteristics. For example, the Tox21 dataset of molecules labeled for their receptor-mediated toxicity contains a mere 10,000 labeled molecules. Processes in accordance with numerous embodiments of the invention are applied to drug discovery and other chemical contexts, where one often has access to many different datasets mapping molecules to different properties (e.g., LogD, toxicity, solubility, membrane permeability, potency against a certain target, etc.), where there can be a wide range of overlap proportions between the different property datasets. Molecule (or drug) candidate properties in accordance with a variety of embodiments of the invention can include physicochemical, biochemical, pharmacokinetic, and pharmacodynamic properties. Examples of properties in accordance with a number of embodiments of the invention can include (but are not limited to) absorption, distribution, metabolism, elimination, toxicity, solubility, metabolic stability, in vivo endpoints, ex vivo endpoints, molecular weight, potency, lipophilicity, hydrogen bonding, permeability, selectivity, pKa, clearance, half-life, volume of distribution, plasma concentration, and stability. Although many of the examples described herein are described with reference to molecular structures, one skilled in the art will recognize that the methods and systems described can be applied to a variety of fields and applications without departing from the invention as defined by the appended claims.

Systems and methods in accordance with a variety of embodiments of the invention treat deep neural networks (DNN's) as differentiable featurizers. In many embodiments, different approaclies are provided for learning accurate mappings from input samples to output labels by exploiting the rich information contained in the intermediate layers of DNNs. In numerous embodiments, training lower variance learners, such as random forests, on an intermediate layer can improve predictive performance compared to a series of subsequent fully connected layers. Deep featurization in accordance with several embodiments of the invention employs a novel technique, referred to as active transfer learning, allowing for more efficient prediction of labels from different data sets or tasks. By training a single master model to predict different tasks (or attributes) based on different data sets, methods in accordance with some embodiments of the invention can generate a master model that can identify relevant and more generalizable features from the inputs, avoiding overfitting to any particular class of data. Other methods for training a model between multiple different tasks include transfer learning and multitask learning. In many cases, transfer learning can be used to train a new model. Transfer learning involves using a model trained for a first task as a starting point for training a model for a different second task. Pre-trained models can provide a large headstart in terms of training time and resources in training of new model. In addition, pre-training can lead to better performance (i.e., more accurate predictions) once training is complete on the desired task. Transfer learning often involves pre-training of a model on one data set and transferring the weights to another model and further training on another data set of interest. Multitask learning involves simultaneous training of a single master neural network that outputs values for all properties for which one has training data.

In some embodiments, deploying active transfer learning, instead of strictly end-to-end differentiable neural network training, can also lead to significant gains in predictive accuracy. Neural networks are known to have a proclivity to overfit the training data. To achieve better generalization performance, or higher accuracy for predicting the properties of molecules that are quite different from those in the training set, one trains a master model, a neural network constituting a series of layers, such as a series of graph convolutional layers and fully connected layers), and, at one or more epochs of training, take the output of one or more of the trained layers to train a composite model (e.g., graph convolution layers + orthogonal learner (e.g., random forest or SVM)). Processes in accordance with various embodiments of the invention can then use as the production model the resulting composite model, with parameters for the composite model selected from the epoch(s) at which the performance on some held-out set of molecules is most accurate. The resulting composite model may exceed the performance of the master model, even if it is only trained on one dataset for one task.

Active transfer learning in accordance with several embodiments of the invention involves a single "deep featurizer" (or master model) to which other task-specific learners (or secondary models) are connected. Systems in accordance with certain embodiments of the invention can be readily applied to a variety of different settings, including (but not limited to) chemical property prediction. In chemical property prediction, one often has access to many (sometimes comparatively small) chemical data sets corresponding to different properties with varying degrees of sample overlap between data sets. Although many of the examples described herein are related to chemical property prediction, one skilled in the art will recognize that similar processes can be applied to a variety of different fields in accordance with different embodiments of the invention. Active transfer learning with deep featurization in accordance with certain embodiments of the invention can improve accuracy on many tasks. There are several possible explanations for the improvement in accuracy. For example, it can at least in part be attributed to the variance reduction wrought by the joint training scheme; the variance reduction wrought by deploying orthogonal models such as random forests which typically have less variance and are less prone to overfitting than deep neural networks; and that sharing weights in the common deep featurizer master model between different datasets / prediction tasks means a richer featurization is learned that can then benefit each of the other tasks individually.

Deep featurizers in accordance with several embodiments of the invention can be used to identify features from data sets. In certain embodiments, deep featurizers can include various different models, including (but not limited to) convolutional neural networks, support vector machines, random forests, ensemble networks, recurrent neural networks, and graph convolution networks. Graph convolutional frameworks in accordance with certain embodiments of the invention treat molecules as graphs and pass information along bonds and space as edges between atoms as nodes, as well as 3D convolutional neural networks. Graph convolution networks are described in greater detail in U.S. Patent Application No. 16/293,586, published as US2019-0272468, entitled "Systems and Methods for Spatial Graph Convolutions with Applications to Drug Discovery and Molecular Simulation," filed on March 5, 2019.

Deep features in accordance with many embodiments of the invention can be exploited in a variety of different ways for learning functions to map a given chemical to various properties.

In the interceding era between logistic regression's preeminence and the rise of deep neural networks, numerous other methods (e.g., random forests, boosting, and support vector machines) came to the fore due to their generally more efficient mapping of fixed input features to the given output. Such methods frequently exceeded the performance of logistic regression. The success of random forests, for example, is thought to stem in part due to the self-regularizing and variance-reducing property of decorrelation between the decision trees, each of which is trained on a random subset of the input features and of the training data. Unfortunately, random forests, boosting, and similar methods cannot be trained end-to-end in a differentiable deep neural network. Whereas deep neural networks are continuous and differentiable functions composed of series of matrix multiplications and pointwise nonlinearities, random forests and boosting cannot be trained with stochastic gradient descent in the same way that DNNs can.

Deep learning has been most successful in realms in which there exists abundantly available training data, while lower variance methods like random forests - when provided with the right features - often outperform neural networks in low data regimes. Methods in accordance with a variety of embodiments of the invention draw on aspects of both approaches that optimize the performance of ML models for settings in which either one or several small data sets are available.

Unlike the domains of vision and natural language, the field of chemical learning faces a relative paucity of available, high-quality, labeled training data. Whereas ImageNet contains $\mathcal{O}(10,000,000)$ labeled images, the Tox21 data set of molecules labeled for their receptor-mediated toxicity contains a mere $\mathcal{O}(10,000)$ labeled molecules.

Multitask learning has been introduced as one way to jointly learn deep neural networks on many smaller data sets to improve performance over separately training many single-task networks. A multitask network maps each input sample (molecule) to many (K) output properties. Multitask learning simultaneously propagates gradient information from the output layer - which outputs predictions for all $K$ tasks - to the input layer.

Transfer learning is an asynchronous relative of multitask learning. Transfer learning involves "pre-training" a neural network on a separate task for which more training data is available, and then transferring the weights as the initialization to a new neural network for the data poorer task of interest.

Ensemble Methods Based on Deep Featurization

In this setting, for a given task and labeled data set associated with that task, steps for a process in accordance with an embodiment of the invention include obtaining features $X$ and labels y and defining neural network $NN$. In various embodiments, the process, for $T$ epochs of end-to-end training of $NN$ to map X to y, will periodically (e.g., every $\frac{T}{E}$ epochs) freeze parameters of $NN$ at epoch $t$ ($NN^{(t)}$), forward propagate X through network, obtain output of layer(s) $h^{(t)}$ from $NN^{(t)}$ (i.e., $h^{(t)}(X)$) and train a non-end-to-end differentiable learner (e.g., random forests), $RF^{(t)}$ Mapping output of layers $h^{(t)}$ to y. The process can then return $NN^{(t)}(X)$ and $RF^{(t)}(X)$ at a single epoch $t$ or a set of epochs $\{e\}$ at which, for example, the validation score(s) is best.

In this example, the process periodically (i.e., every T/E epochs) freezes the parameters of the master model and propagates a set of inputs through the network to compute features for the inputs at layer(s) $h^{(t)}$ in order to train an orthogonal learner to map the computed features to the labels y. In numerous embodiments, the orthogonal model and/or deep featurizer are validated at each T/E epochs, and the orthogonal model and/or deep featurizer at the optimal epoch are selected to build a composite model with the deep featurizer generating features for the orthogonal model.

Specific processes for active transfer learning in accordance with embodiments of the invention are described above; however, one skilled in the art will recognize that any number of processes can be utilized as appropriate to the requirements of specific applications in accordance with embodiments of the invention as defined by the appended claims.

Neural network training with both train and valid data

Several ensemble methods, including random forests, have an "out of bag" score or equivalent that enables one to monitor the generalization performance of the sub-decision trees within the model on data held out from each of the trees. This confers the advantage of the final model being trained on all available training data without needing a held-out validation set that is disjoint from the training or test sets to avoid overfitting. Analogous procedures for training-while-validating on the same data set do not exist in the realm of deep neural networks. Typically, in the context of DNN training, disjoint training, validation, and test data subsets are defined, gradient information is derived from the training set to optimize the weights of the neural network, and performance on the validation set is used for early stopping and model selection.

In various embodiments, the "out of bag" error can also be used as an early stopping criterion for neural networks that enables one to train-while-validating on a concatenation of the training and validation sets. An example process in accordance with a variety of embodiments of the invention can obtain features X and labels y and define neural network $NN$. In a number of embodiments, the process can, for $T$ epochs of end-to-end training of $NN$ to map X to $y$, periodically (e.g., every $\frac{T}{E}$ epochs), freeze parameters of $NN$ at epoch $t$ ($NN^{(t)}$), forward propagate X through network, obtain output of layer(s) $h^{(t)}$ for convenience) from $NN^{(t)}$, train an ensemble learner (e.g., random forests), $RF^{(t)}$ Mapping $h^{(t)}$ to y, and record out-of-bag score at epoch $t$. The process can then return $NN^{(t)}$ and $RF^{(t)}$ at epoch $t$ at which the out-of-bag score is best.

In some embodiments, what are typically delineated as the training and validation sets can both be used for both the training and validation of a neural network. For example, for features X and labels y, processes in accordance with a number of embodiments of the invention can, for $T$ epochs, perform end-to-end training of $[X_{train}, X_{valid}]$ and $[y_{train}, y_{valid}]$ concatenated together. In several embodiments, processes can periodically freeze parameters of $NN$ and train an ensemble learner (e.g., random forests) on only the training data to map $X^{(train)}$ to $y^{(train)}$. Processes in accordance with certain embodiments of the invention can make predictions for $X^{(valid)}$ to obtain $\hat{y}^{(valid)}$, and compute a validation score by comparing $\hat{y}^{(valid)}$ with $y^{(valid)}$.

Active Transfer Learning with Deep Featurization

Transfer learning entails training a DNN on a task with a (typically) large data set and transferring the resulting parameters as an initialization to a new DNN to be trained on a new task and associated data set of interest. In contrast, multitask learning entails simultaneous learning of a single "master" network that outputs predictions for all desired tasks. Transfer learning can be effective in scenarios even where there is little to no overlap between the training samples in the different data sets/tasks. In contrast, multitask learning is best applied in scenarios where there is substantial (ideally, full) overlap between the training samples in the different data sets/tasks. When there is either little overlap between the data sets or little correlation between the tasks, multitask learning can actually reduce, rather than improve, the performance of DNNs. In general, if one imagines the training labels y as a large NxK matrix where N is the total number of training samples and K is the number of tasks, the sparser the matrix or the less correlated the columns leads to a diminished, or in some cases, a counterproductive, multitask effect.

In drug discovery and other chemical contexts, one often has access to many different data sets mapping molecules to different properties (e.g., LogD, toxicity, solubility, membrane permeability, potency against a certain target), with a wide range of overlap proportions between the different property data sets. Active transfer learning with deep featurization has been shown to address such problems. An example of a procedure for active transfer learning is provided below.

In this example, a process in accordance with several embodiments of the invention can define master featurizer neural network $NN^{(f)}$. The process can then, for each task $k$ of all $K$ tasks/data sets (or single task/dataset), define sub neural network $NN^{(k)}$, and obtain features $X^{(k)}$ and labels $y^{(k)}$. Then, for $T$ epochs and for each task k of all K task/data sets, the process in accordance with several embodiments of the invention can link $NN^{(f)}$ with $NN^{(k)}$ to form $NN^{[f,k]}$ and train $NN^{[f,k]}$ for one epoch with $(X^{(k)}, y^{(k)})$. Periodically (e.g., when epoch $t$ is a multiple of $\frac{T}{E}$), the process can freeze parameters of $NN^{(f)}$ at epoch $t$: $NN^{ft}$, forward propagate X through network $NN^{ft}$, obtain output of layer(s) $h^{(k,t)}$ from $NN^{(ft)}$, and train an ensemble learner (e.g., random forests), $RF^{(k,t)}$ Mapping $h^{(k,t)}(X)$ to $y^{(k)}(X)$. The process can then return set $\{NN^{(k,t)}\}$ and set $\{RF^{(k,t)}\}$ for each task $k$ at epochs $t_k$ at which validation score(s) are optimal.

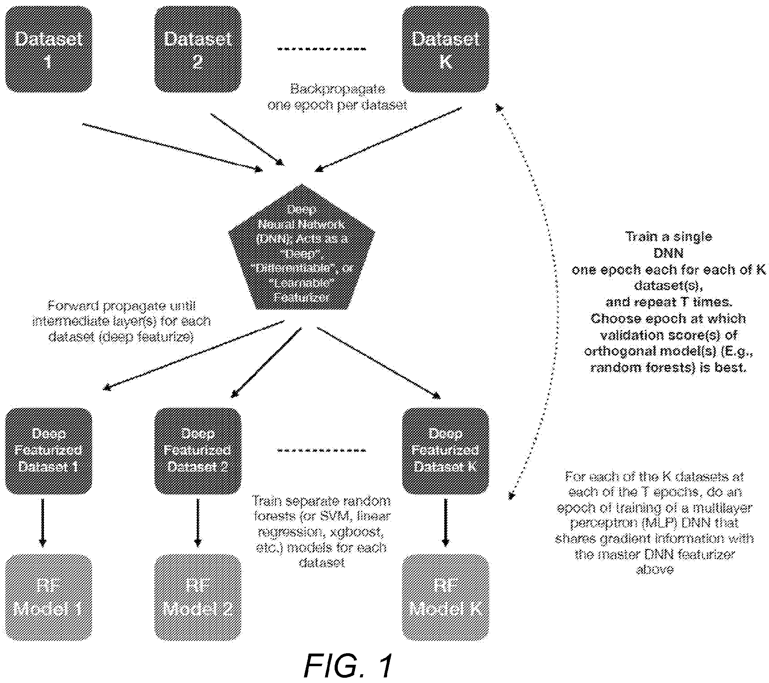

An illustration of the method is provided in Figure 1. Figure 1 shows data set(s) 1-K, which are used to train a single featurizer DNN (e.g., PotentialNet or another graph convolutional neural network) across a number of epochs. Every epoch of training entails training an epoch for each individual data set, each of which has its own fully connected layers which pass gradient information through the deep featurizer back to the input. The layers are then frozen and the data is forward propagated to generate deep featurized data set(s) 1-K. Separate models (e.g., random forests, SVM, linear regression, xgboost, etc.) are then trained for each deep featurized data set. The epoch at which an aggregate validation score (e.g., an average OOB score) is best is selected for the final model. In numerous embodiments, for each of the K dataset(s) at each of the Tepochs, processes can perform an epoch of training of a multilayer perceptron (MLP) DNN that shares gradient information with the master DNN featurizer.

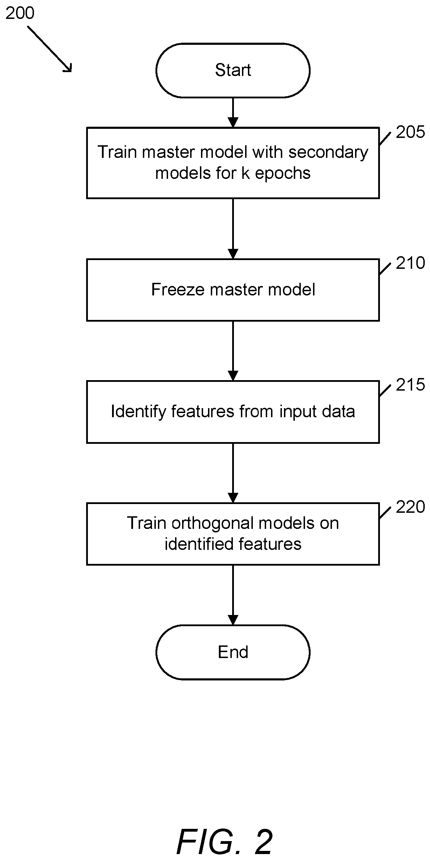

An active transfer learning process in accordance with an embodiment of the invention is shown in FIG. 2.

Process 200 trains (205) a master model with secondary models for a number of epochs. Secondary models can each train the master model for different sets of labels. In a variety of embodiments, the number of epochs can be a set number of epochs or a random number of epochs. In a number of embodiments, a number of datasets is trained in each epoch, where each dataset trains the model on a different subset of labels or properties. Process 200 freezes (210) the weights of the master model. Input data is then processed through the master model to identify (215) features from the input data. Identified features in accordance with a number of embodiments of the invention include feature vectors and other feature descriptors. Process 200 then trains (220) orthogonal models on the identified features. Orthogonal models in accordance with various embodiments of the invention can include non-differentiable ensemble models, such as (but not limited to) random forests. In certain embodiments, the combination of the featurizer and a set of one or more orthogonal model are used together to predict or classify inputs.

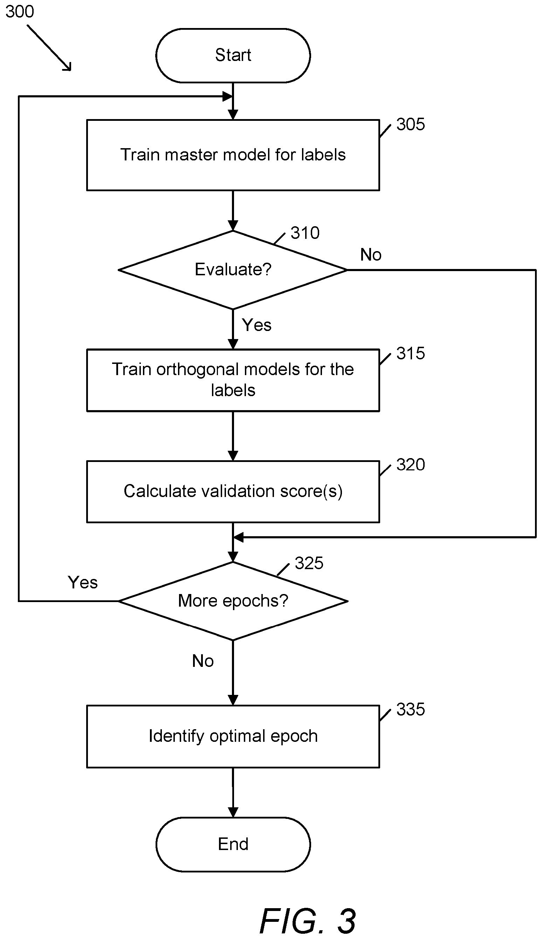

An active transfer learning process in accordance with an embodiment of the invention is shown in FIG. 3. Process 300 trains (305) a master model for one or more labels across one or more data sets. Process 300 then determines (310) whether to evaluate the model. In various embodiments, processes can determine to evaluate the model after a set number of epochs. Processes in accordance with certain embodiments of the invention can determine to evaluate the model in a random fashion. When process 300 determines to evaluate the model, the process trains (315) one or more orthogonal models for the labels. In some embodiments, a separate orthogonal model is trained to classify for each label and/or data set. In this way, processes in accordance with various embodiments of the invention train a hybrid model consisting of a deep neural network acting as a featurizer with another learner that makes the final prediction mapping the features of each input sample to the output property of interest. Process 300 calculates (320) one or more validation scores for the master model and/or the orthogonal models. Validation scores in accordance with a variety of embodiments of the invention can include (but are not limited to) "out of bag" errors and validation scores for the model based on a validation set picked from a data set. Process 300 then determines (325) whether there are more epochs to perform. If so, process 300 returns to step 305. When process determines (325) that no more epochs are to be performed, the process identifies (335) an optimal epoch. In a variety of embodiments, optimal epochs are identified based on an aggregate validation score, such as (but not limited to) an average, a maximum, etc. In a variety of embodiments, the optimal epochs can then be used to produce a composite model. Processes in accordance with certain embodiments of the invention can build a composite model using a combination of the weighted layers of the master model and the trained orthogonal model at the optimal epoch.

Specific processes for active transfer learning in accordance with embodiments of the invention are described above; however, one skilled in the art will recognize that any number of processes can be utilized as appropriate to the requirements of specific applications in accordance with embodiments of the invention as defined by the appended claims.

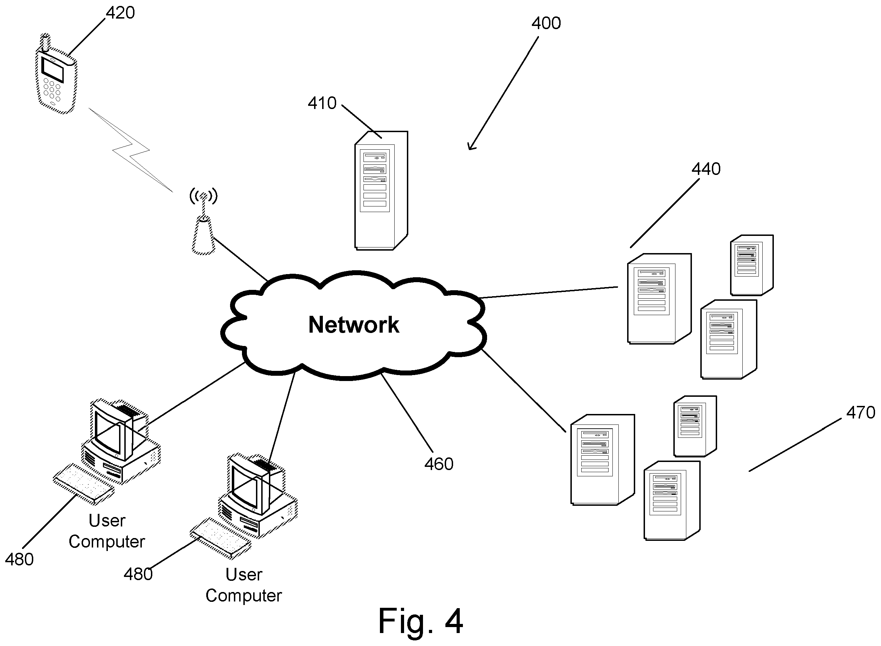

A system that trains machine learning models in accordance with some embodiments of the invention is shown in Fig. 4. Network 400 includes a communications network 460. The communications network 460 is a network such as the Internet that allows devices connected to the network 460 to communicate with other connected devices. Server systems 410, 440, and 470 are connected to the network 460. Each of the server systems 410, 440, and 470 is a group of one or more servers communicatively connected to one another via internal networks that execute processes that provide cloud services to users over the network 460. For purposes of this discussion, cloud services are one or more applications that are executed by one or more server systems to provide data and/or executable applications to devices over a network. The server systems 410, 440, and 470 are shown each having three servers in the internal network. However, the server systems 410, 440 and 470 may include any number of servers and any additional number of server systems may be connected to the network 460 to provide cloud services. In accordance with various embodiments of this invention, a deep learning network that uses systems and methods that train master and orthogonal models in accordance with an embodiment of the invention may be provided by a process being executed on a single server system and/or a group of server systems communicating over network 460.

Users may use personal devices 480 and 420 that connect to the network 460 to perform processes for providing and/or interaction with a deep learning network in accordance with various embodiments of the invention. In the shown embodiment, the personal devices 480 are shown as desktop computers that are connected via a conventional "wired" connection to the network 460. However, the personal device 480 may be a desktop computer, a laptop computer, a smart television, an entertainment gaming console, or any other device that connects to the network 460 via a "wired" connection. The mobile device 420 connects to network 160 using a wireless connection. A wireless connection is a connection that uses Radio Frequency (RF) signals, Infrared signals, or any other form of wireless signaling to connect to the network 460. In Figure 4, the mobile device 420 is a mobile telephone. However, mobile device 420 may be a mobile phone, Personal Digital Assistant (PDA), a tablet, a smartphone, or any other type of device that connects to network 460 via wireless connection without departing from this invention as defined by the appended claims.

Model Training Element

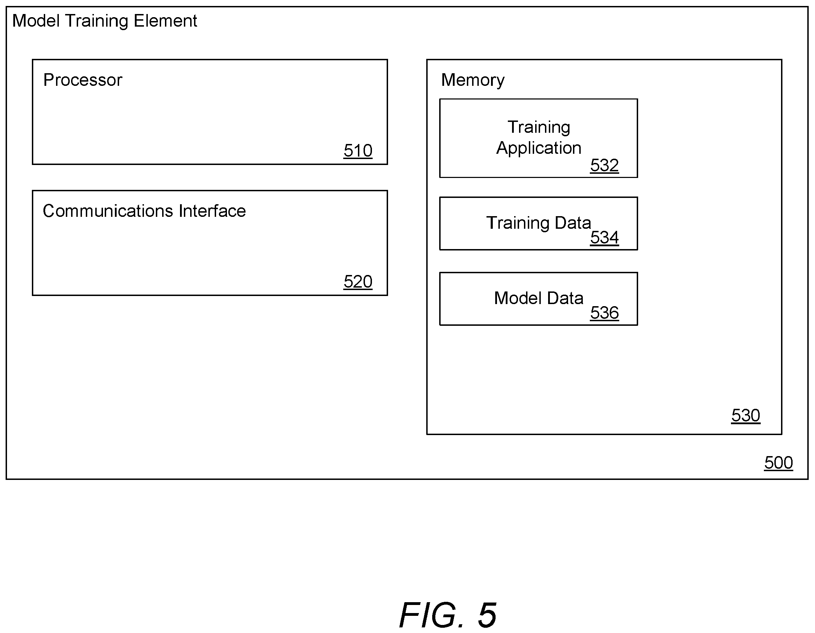

An example of a model training element that executes instructions to perform processes that train master and orthogonal models with other devices connected to a network and/or for providing training tasks in accordance with various embodiments of the invention is shown in Figure 5. Training elements in accordance with many embodiments of the invention can include (but are not limited to) one or more of mobile devices, computers, servers, and cloud services. Training element 500 includes processor 510, communications interface 520, and memory 530.

One skilled in the art will recognize that a particular training element may include other components that are omitted for brevity without departing from this invention as defined by the appended claims. The processor 510 can include (but is not limited to) a processor, microprocessor, controller, or a combination of processors, microprocessor, and/or controllers that performs instructions stored in the memory 530 to manipulate data stored in the memory. Processor instructions can configure the processor 510 to perform processes in accordance with certain embodiments of the invention. Communications interface 520 allows training element 500 to transmit and receive data over a network based upon the instructions performed by processor 510.

Memory 530 includes a training application 532, training data 534, and model data 536. Training applications in accordance with several embodiments of the invention are used to train a featurizer through the training of master models, secondary models, and orthogonal models. Featurizers in accordance with a number of embodiments of the invention are composite models composed of a master model and one or more orthogonal models that use features of the inputs to predict a number of different characteristics of the inputs. In several embodiments, training applications can train a featurizer model to identify generalizable and relevant features of an input class (e.g., chemical compounds). Training application in accordance with certain embodiments of the invention can use training data to train one or more master models, secondary models, and orthogonal models to determine an optimized featurizer for featurizing a set of inputs.

Although a specific example of a training element 500 is illustrated in Figure 5, any of a variety of training elements can be utilized to perform processes similar to those described herein as appropriate to the requirements of specific applications in accordance with embodiments of the invention as defined by the appended claims.

Training Application

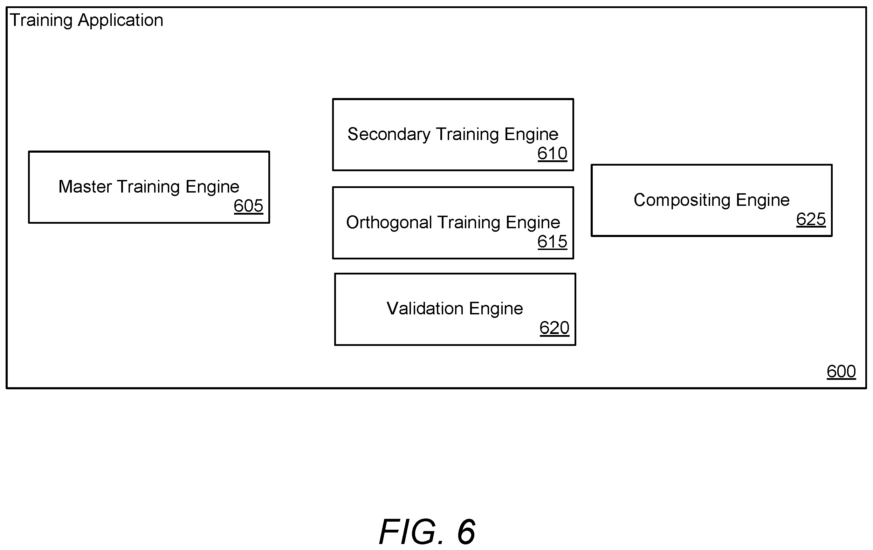

A training application for training deep featurizers in accordance with an embodiment of the invention is illustrated in Figure 6. Training application 600 includes master training engine 605, secondary training engine 610, orthogonal training engine 615, validation engine 620, and compositing engine 625. Training applications in accordance with many embodiments of the invention train a deep featurizer on a limited set of training data to predict or classify new inputs across a number of different labels.

In a variety of embodiments, master training engines can be used to train a master model to identify generalizable features from input data across multiple classes or tasks. In many embodiments, a master model and a set of one or more orthogonal models make up a composite model that is able to use broadly generalizable features to classify new inputs.

Secondary training engines in accordance with a variety of embodiments of the invention can be used to train secondary models for training a master model on a set of data. In some embodiments, secondary training engines use a classifier (such as, but not limited to, fully connected layers) to compute a loss that can be back propagated through the master model. In several embodiments, a separate secondary model is trained for each of a plurality of different data sets, allowing the master model to be trained across multiple different label sets. For example, in some embodiments each data set is associated with a set of one or more properties (such as, but not limited to LogD, toxicity, solubility, membrane permeability, potency against a certain target), and a different secondary model is trained for each set of properties.

Orthogonal training engines in accordance with many embodiments of the invention can be used to train orthogonal models for training a master model. In many embodiments, orthogonal models can include (but are not limited to) random forests and support vector machines. Orthogonal models in accordance with a number of embodiments of the invention can be trained on layers of the master model during training and to provide an orthogonal loss for adjusting the weights of the master model.

Validation engines in accordance with numerous embodiments of the invention are used to validate the results of orthogonal models and/or master models to determine an optimized stopping point for the master and/or orthogonal models. In a variety of embodiments, validation engines can compute out of bag errors to to monitor the generalization performance of the models, allowing for the selection of optimal weights for a composite model.

In a variety of embodiments, compositing engines can generate a composite model as a deep featurizer based on training processes and systems described above. Composite models in accordance with certain embodiments of the invention include a master model and a set of one or more orthogonal models. The master model and the set of orthogonal models can be weighted based on a set of weights for which a validation score (such as, but not limited to, an out of bag score) is best.

Although a specific example of a training application is illustrated in Figure 6, any of a variety of training applications can be utilized to perform processes similar to those described herein as appropriate to the requirements of specific applications in accordance with embodiments of the invention as defined by the appended claims.

Results

The methods described in this description have been validated with both publicly available datasets as well as proprietary massive pharmaceutical datasets. In this section, results for model performance on three publicly available chemical datasets (ESOL (Solubility), SAMPL (Solubility), and Lipophilicity) are provided. Since random splitting is widely believed to overestimate the real-world performance of chemical machine learning models, a form of scaffold splitting (K-Means clustering of chemical samples projected onto circular fingerprint space) is used for this example. The table below shows that, for each dataset, joint training with active transfer learning in accordance with some embodiments of the invention outperforms training with graph convolution PotentialNet alone.

| Model | ESOL $R^2$ | SAMPL $R^2$ | Lipophilicity $R^2$ |
|---|---|---|---|
| PotentialNet Alone | 0.368 | 0.827 | 0.521 |
| Active Transfer Learning with PotentialNet as Featurizer | **0.467** | **0.923** | **0.567** |

Although the present invention has been described in certain specific aspects, many additional modifications and variations would be apparent to those skilled in the art. It is therefore to be understood that the present invention may be practiced otherwise than specifically described.

**Claims**

1.   A computer-implemented method for drug discovery, the method comprising:

(a) collecting one or more datasets of one or more molecules, wherein each dataset of the one or more datasets has labels for a different characteristic of inputs of the dataset;
(b) training a deep featurizer, wherein training the deep featurizer comprises:

(i) training a master model and a set of one or more secondary models based on the one or more datasets, wherein the master model is a neural network that comprises a set of one or more layers, and training the master model based on the trained secondary models,
(ii) creating a set of outputs from the master model, and
(iii) training a set of one or more orthogonal models on the created set of outputs;

(c) creating a feature set of one or more outputs from the deep featurizer; and
(d) identifying a drug candidate using the trained set of one or more orthogonal models.

2.   The method of claim 1, further comprising, prior to (b)(ii), freezing weights of the master model.

3.   The method of claims 1 or 2, wherein training the master model comprises training the master model for one or more epochs.

4.   The method of any one of claims 1 to 3, wherein each epoch comprises training the master model and the set of secondary models on one or more datasets.

5.   The method of any one of claims 1 to 4, wherein creating the set of outputs comprises propagating the one or more datasets through the master model..

6.   . The method of any one of the preceding claims, further comprising validating the master model and the set of one or more orthogonal models.

7.   The method of claim 6, wherein validating the set of one or more orthogonal models comprises computing an out of bag score for the set of one or more orthogonal models.

8.   The method of claim 6, wherein validating the set of one or more orthogonal models comprises:

(1) training the master model on a master data set comprising a training data set and a validation data set;

(2) training the set of one or more orthogonal models on the training data set; and
(3) computing a validation score for the one or more orthogonal models based on the validation data set.

9. The method of any one of the preceding claims, wherein the set of one or more orthogonal models comprises at least one of random forest, a support vector machine, XGBoost, linear regression, nearest neighbor, naive bayes, decision trees, neural networks, and k-means clustering.

10. The method of any one of the preceding claims, wherein training the master model comprises training the master model for one or more epochs, and further comprising:
compositing the master model and the set of one or more orthogonal models as a composite model to classify a new set of inputs, wherein the compositing is based on using a combination of weighted layers of the master model and the trained orthogonal models at an optimal epoch, wherein the optimal epoch is identified based on an aggregate validation score.

11. The method of any one of the preceding claims, wherein

the trained master model or trained one or more orthogonal models predict a property of the drug candidate; and
the property of the drug candidate comprises at least one of the group consisting of absorption, distribution, metabolism, elimination, toxicity, solubility, metabolic stability, in vivo endpoints, ex vivo endpoints, molecular weight, potency, lipophilicity, hydrogen bonding, permeability, selectivity, pKa, clearance, half-life, volume of distribution, plasma concentration, and stability.

12. The method of any one of the preceding claims, wherein the one or more molecules is a ligand molecule and/ or a target molecule.

13. The method of claim 12, wherein the target molecule is a protein.

14. A non-transitory machine-readable medium containing processor instructions that, when executed by one or more processors, cause the one or more processors to carry out the method of any one of claims 1 to 13.

15. A system comprising:

one or more processors; and
one or more storage devices communicatively coupled to the one or more processors, wherein the one or more storage devices store instructions that, when executed by the one or more processors, cause the one or more processors to carry out the method of any one of claims 1 to 13.

**Patentansprüche**

1. Computer-implementiertes Verfahren zur Arzneimittel-Discovery, wobei das Verfahren umfasst:

(a) Sammeln eines oder mehrerer Datensätze von einem oder mehreren Molekülen, wobei jeder Datensatz des bzw. der Datensätze Kennungen für ein verschiedenes Merkmal von Eingaben des Datensatzes aufweist;
(b) Trainieren eines Deep-Featurizers, wobei das Trainieren des Deep Featurizer umfasst:

(i) Trainieren eines Mastermodells und einer Menge von einem oder mehreren Sekundärmodellen auf Grundlage des bzw. der Datensätze, wobei das Mastermodell ein neuronales Netzwerk ist, das eine Menge aus einer oder mehreren Schichten umfasst, und
Trainieren des Mastermodells auf Grundlage der trainierten Sekundärmodelle,
(ii) Erzeugen einer Menge von Ausgaben des Mastermodells, und
(iii) Trainieren einer Menge von einem oder mehreren orthogonalen Modellen auf der erzeugten Menge von Ausgaben;

(c) Erzeugen einer Merkmalsmenge von einem oder mehreren Ausgängen des Deep Featurizers; und
(d) Identifizieren eines Arzneimittelkandidaten mittels der trainierten Menge von einem oder mehreren orthogonalen Modellen.

**2.** Verfahren nach Anspruch 1, zudem umfassend vor (b)(ii) das Einfrieren der Gewichte des Master-Modells.

**3.** Verfahren nach Anspruch 1 oder 2, wobei das Trainieren des Master-Modells das Trainieren des Master-Modells für eine oder mehrere Epochen umfasst.

**4.** Verfahren nach einem der Ansprüche 1 bis 3, wobei jede Epoche das Trainieren des Master-Modells und der Menge von Sekundärmodellen auf einem oder mehreren Datensätzen umfasst.

**5.** Verfahren nach einem der Ansprüche 1 bis 4, wobei das Erzeugen der Menge von Ausgaben das Propagieren des bzw. der Datensätze durch das Mastermodell umfasst.

**6.** Verfahren nach einem der vorhergehenden Ansprüche, zudem umfassend Validieren des Mastermodells und der Menge von einem oder mehreren orthogonalen Modellen.

**7.** Verfahren nach Anspruch 6, wobei das Validieren der Menge von einem oder mehreren orthogonalen Modellen das Berechnen eines Out-of-Bag-Scores für die Menge von einem oder mehreren orthogonalen Modellen umfasst.

**8.** Verfahren nach Anspruch 6, wobei das Validieren der Menge von einem oder mehreren orthogonalen Modellen umfasst:

(1) Trainieren des Mastermodells auf einem Masterdatensatz, der einen Trainingsdatensatz und einen Validierungsdatensatz umfasst;
(2) Trainieren der Menge von einem oder mehreren orthogonalen Modellen auf dem Trainingsdatensatz; und
(3) Berechnen eines Validierungsergebnisses für das bzw. die orthogonale(n) Modell(e) auf Grundlage des Validierungsdatensatzes.

**9.** Verfahren nach einem der vorhergehenden Ansprüche, wobei die Menge von einem oder mehreren orthogonalen Modellen zumindest eines umfasst von: Random Forest, einer Support-Vektor-Maschine, XGBoost, linearer Regression, nächsten Nachbarn, Naiver Bayes, Entscheidungsbäumen, neuronalen Netzen und k-means-Clustering.

**10.** Verfahren nach einem der vorhergehenden Ansprüche, wobei das Trainieren des Master-modells das Trainieren des Mastermodells für eine oder mehrere Epochen umfasst und zudem umfasst:
Zusammenstellen des Mastermodells und der Menge von einem oder mehreren orthogonalen Modellen als ein zusammengesetztes Modell zum Klassifizieren einer neuen Menge von Eingaben, wobei das Zusammenstellen auf der Verwendung einer Kombination von gewichteten Schichten des Mastermodells und der trainierten orthogonalen Modelle in einer optimalen Epoche basiert, wobei die optimale Epoche auf der Grundlage eines aggregierten Validierungsergebnisses bestimmt wird.

**11.** Verfahren nach einem der vorhergehenden Ansprüche, wobei

das trainierte Mastermodell oder das bzw. die trainierten orthogonale Modelle eine Eigenschaft des Arzneimittelkandidaten vorhersagen; und
die Eigenschaft des Arzneimittelkandidaten zumindest eine umfasst aus der Gruppe bestehend aus Absorption, Verteilung, Metabolismus, Eliminierung, Toxizität, Löslichkeit, metabolische Stabilität, in-vivo Endpunkte, ex-vivo Endpunkte, Molekulargewicht, Potenz, Lipophilie, Wasserstoffbrückenbindung, Permeabilität, Selektivität, pKa, Clearance, Halbwertszeit, Verteilungsvolumen, Plasmakonzentration und Stabilität.

**12.** Verfahren nach einem der vorhergehenden Ansprüche, wobei das bzw. die Moleküle ein Ligandenmolekül und/oder ein Zielmolekül sind.

**13.** Verfahren nach Anspruch 12, wobei das Zielmolekül ein Protein ist.

**14.** Nicht-transitorisches maschinenlesbares Medium, enthaltend Prozessorbefehle, die bei Ausführung durch einen oder mehrere Prozessoren diese(n) veranlassen, das Verfahren nach einem der Ansprüche 1 bis 13 durchzuführen.

**15.** System, umfassend:

einen oder mehrere Prozessoren; und

eine oder mehrere Speichervorrichtungen, die kommunikativ mit dem bzw. den Prozessoren gekoppelt sind, wobei die Speichervorrichtung(en) Anweisungen speichern, die bei Ausführung durch den bzw. die Prozessoren diese(n) veranlassen, das Verfahren nach einem der Ansprüche 1 bis 13 durchzuführen.

## Revendications

1. Procédé de découverte de médicaments mise en œuvre par ordinateur, comprenant :

    (a) collecter un ou plusieurs ensembles de données d'une ou de plusieurs molécules, chaque ensemble de données du ou des ensembles de données ayant des termes pour une caractéristique différente des entrées de l'ensemble de données ;
    (b) entraîner un featuriser profond, ledit entraînement comportant :

    (i) entraîner un modèle maître et un ensemble d'un ou plusieurs modèles secondaires sur la base d'un ou de plusieurs ensembles de données, ledit modèle maître étant un réseau neuronal qui comprend un ensemble d'une ou plusieurs couches, et
    entraîner le modèle maître sur la base des modèles secondaires formés,
    (ii) créer un ensemble de sorties du modèle maître, et
    (iii) former un ensemble d'un ou de plusieurs modèles orthogonaux sur l'ensemble de sorties créé ;

    (c) créer un ensemble de caractéristiques d'une ou plusieurs sorties du featuriser profond ; et
    (d) identifier un candidat-médicament à l'aide de l'ensemble formé d'un ou de plusieurs modèles orthogonaux.

2. Procédé de la revendication 1, comprenant en outre, avant (b)(ii), congeler les poids du modèle maître.

3. Procédé des revendications 1 ou 2, dans lequel entraîner le modèle maître comprend entraîner le modèle maître pendant une ou plusieurs époques.

4. Procédé de l'une quelconque des revendications 1 à 3, dans lequel chaque époque comprend entraîner le modèle maître et l'ensemble des modèles secondaires sur un ou plusieurs ensembles de données.

5. Procédé de l'une quelconque des revendications 1 à 4, dans lequel créer l'ensemble de sorties comprend la propagation d'un ou de plusieurs ensembles de données à travers le modèle maître.

6. Procédé de l'une quelconque des revendications précédentes, comprenant en outre la validation du modèle maître et de l'ensemble d'un ou de plusieurs modèles orthogonaux.

7. Procédé de la revendication 6, dans lequel la validation de l'ensemble d'un ou de plusieurs modèles orthogonaux comprend le calcul d'un score « out-of-bag » pour l'ensemble d'un ou de plusieurs modèles orthogonaux.

8. Procédé de la revendication 6, dans lequel la validation de l'ensemble d'un ou de plusieurs modèles orthogonaux comprend :

    (1) entraîner le modèle maître sur un ensemble de données principal qui comprend un ensemble de données d'entraînement et un ensemble de données de validation ;
    (2) entraîner l'ensemble d'un ou plusieurs modèles orthogonaux sur l'ensemble de données d'entraînement ; et
    (3) calculer un score de validation pour le ou les modèles orthogonaux sur la base de l'ensemble de données de validation.

9. Procédé de l'une quelconque des revendications précédentes, dans lequel l'ensemble d'un ou de plusieurs modèles orthogonaux comprend au moins l'un des éléments suivants : forêt aléatoire, machine à vecteurs de support, XGBoost, régression linéaire, plus proche voisin, bayes naïves, arbres de décision, réseaux neuronaux et regroupement par k-moyennes.

10. Procédé de l'une quelconque des revendications précédentes, dans lequel l'entraînement du modèle maître comprend l'entraînement du modèle maître pendant une ou plusieurs époques, et comprend en outre :
    compositer le modèle maître et l'ensemble d'un ou plusieurs modèles orthogonaux en tant que modèle composite pour classer un nouvel ensemble d'entrées, ledit compositer étant basé sur l'utilisation d'une combinaison de couches pondérées du modèle maître et des modèles orthogonaux entraînés à une époque optimale, ladite époque optimale étant identifiée sur la base d'un score de validation agrégé.

11. Procédé de l'une quelconque des revendications précédentes, dans lequel

le modèle maître entraîné ou un ou plusieurs modèles orthogonaux entraînés prédisent une propriété du candidat-médicament ; et
la propriété du candidat-médicament comporte au moins l'un des éléments suivants : absorption, distribution, métabolisme, élimination, toxicité, solubilité, stabilité métabolique, paramètres in vivo, paramètres ex vivo, poids moléculaire, puissance, lipophilie, liaison hydrogène, perméabilité, sélectivité, pKa, clairance, demi-vie, volume de distribution, concentration plasmatique, et stabilité.

12. Procédé de l'une quelconque des revendications précédentes, dans lequel la ou les molécules sont une molécule ligand et/ou une molécule cible.

13. Procédé de la revendication 12, dans lequel la molécule cible est une protéine.

14. Support non transitoire lisible à la machine contenant des instructions de processeur qui, lorsqu'elles sont exécutées par un ou plusieurs processeurs, amènent ledit ou lesdits processeurs à mettre en œuvre la procédé de l'une des revendications 1 à 13.

15. Système comprenant :

un ou plusieurs processeurs ; et
un ou plusieurs dispositifs de stockage couplés de manière communicative audit ou auxdits processeurs, ledit ou lesdits dispositifs de stockage mémorisant des instructions qui, lorsqu'elles sont exécutées par ledit ou lesdits processeurs, amènent ledit ou lesdits processeurs à mettre en œuvre la procédé de l'une des revendications 1 à 13.

Dataset 1

Dataset 2

Dataset K

Backpropagate one epoch per dataset

Deep Neural Network (DNN) Act as a Deep Featurizer or Learnable Featurizer

Forward propagate until intermediate layer(s) for each dataset (deep featurize)

Train a single DNN one epoch each for each of K dataset(s), and repeat T times. Choose epoch at which validation score(s) of orthogonal model(s) (E.g., random forests) is best.

For each of the K datasets at each of the T epochs, do an epoch of training of a multilayer perceptron (MLP) DNN that shares gradient information with the master DNN featurizer above

Deep Featurized Dataset K

Deep Featurized Dataset 2

Deep Featurized Dataset 1

Train separate random forests (or SVM, linear regression, xgboost, etc.) models for each dataset

RF Model K

RF Model 2

RF Model 1

*FIG. 1*

200

Start

Train master model with secondary
models for k epochs

205

Freeze master model

210

Identify features from input data

215

Train orthogonal models on
identified features

220

End

FIG. 2

300

FIG. 3

Fig. 4

EP 3 871 154 B1

Model Training Element

Processor

510

Communications Interface

520

Memory

Training Application
532

Training Data
534

Model Data
536

530

500

FIG. 5

Training Application

Master Training Engine
605

Secondary Training Engine
610

Orthogonal Training Engine
615

Validation Engine
620

Compositing Engine
625

600

EP 3 871 154 B1

*FIG. 6*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 293586

- US 20190272468 A

**Non-patent literature cited in the description**

- A Combination of Feature Extraction Methods with an Ensemble of Different Classifiers for Protein Structural Class Prediction Problem. **DEHZANGI AB-DOLLAH et al.** IEEE/ACM Transactions On Computational Biology And Bioinformatics. IEEE Service Center, 01 May 2013, vol. 10, 564-575